(19) Europäisches Patentamt
European Patent Office
Office européen des brevets

(11) Numéro de publication: **0 188 169**
**B1**

(12) # FASCICULE DE BREVET EUROPEEN

(45) Date de publication du fascicule du brevet: **27.01.88**

(51) Int. Cl.⁴: **C 07 C 93/00,** A 61 K 31/00

(21) Numéro de dépôt: **85450028.7**

(22) Date de dépôt: **20.11.85**

(54) **Bis-((hydroxy-4 isopropyl-2 méthyl-5 phénoxy)-2 amines, leur méthode de synthèse et les préparations pharmaceutiques les contenant.**

(30) Priorité: **12.12.84 FR 8419103**

(43) Date de publication de la demande: **23.07.86 Bulletin 86/30**

(45) Mention de la délivrance du brevet: **27.01.88 Bulletin 88/4**

(84) Etats contractants désignés: **BE CH DE GB IT LI LU NL**

(56) Documents cités: **EP - A - 0 062 596**

(73) Titulaire: **SOCIETE CORTIAL S.A., 7 rue de l'Armorique, F-75015 Paris (FR)**

(72) Inventeur: **Greuzet, Marie-Hélène, Résidence Jardin de Gambetta T 3, F-33000 Bordeaux (FR)**
Inventeur: **Feniou, Claude, 5 rue du Général Guillaumat, F-33600 Pessac (FR)**
Inventeur: **Goussot, Gérard, Cité Bois de Mont 15 rue du Dr Férié, F-33160 St Medard en Jalles (FR)**
Inventeur: **Prat, Gisèle, Hameau de Noailles Villa 18, F-33400 Talence (FR)**
Inventeur: **Pontagnier, Henri, 21 rue Edouard Vaillant, F-33600 Pessac (FR)**

(74) Mandataire: **Tajan, Marie-Thérèse, LABORATOIRES SARGET Avenue du Président JF Kennedy, F-33701 Mérignac (FR)**

**EP 0 188 169 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

ACTORUM AG

## Description

La présente invention concerne des *bis*-((hydroxy-4 isopropyl-2 méthyl-5 phénoxy)-2 éthyl) amines, leur méthode de synthèse, les préparations pharmaceutiques les contenant et leur application en thérapeutique.

Ces nouveaux produits ont pour formule générale

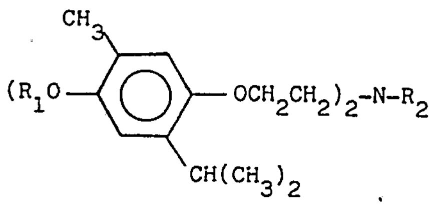
(I)

dans laquelle $R_1$ représente H, $CH_3$ ou $COCH_3$ et $R_2$ représente H ou $CH_3$. Ces nouveaux produits peuvent être sous forme de base libre ou de sels pharmaceutiquement compatibles tels que les sels d'addition d'acides minéraux (par exemple acide chlorhydrique, bromhydrique, sulfurique, phosphorique) ou organiques (par exemple acides citrique, méthanesulfonique, camphosulfonique).

On connaît déjà des dérivés de l'(hydroxy-4 isopropyl-2 méthyl-5 phénoxy)-2 éthylamine utiles en thérapeutique. Ainsi, le moxisylyte, produit de formule

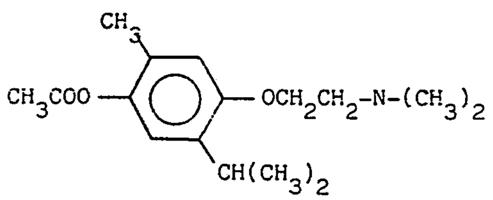
(II)

est utilisé en thérapeutique dans le traitement des troubles psychocomportementaux de la sénescence, des troubles cochléovestibulaires, des déficits circulatoires rétiniens, de certains troubles circulatoires des extrémités ou acrosyndromes, et des perturbations vasculaires du début de la ménopause.

Le brevet européen N° 62596 déposé le 01.04.1982 décrit des dérivés du moxisylyte de formule générale

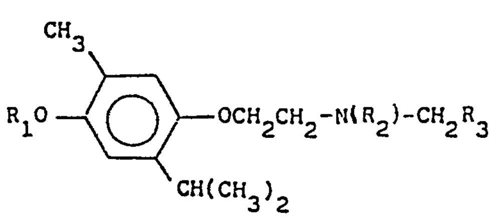
(III)

dans laquelle $R_1$ représente H, $CH_3$ ou $COCH_3$, $R_2$ représente H ou $CH_3$ et $R_3$ représente $CH_2-O-C_6H_5$,

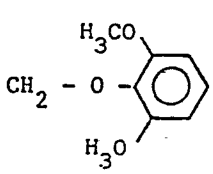
, ou
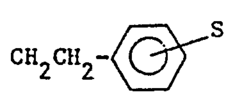

avec S = H ou un ou plusieurs substituants tels que OH, $OCH_3$ ou halogène.

Les produits décrits dans le brevet européen 62596 présentent des propriétés alphabloquantes postsynaptiques beaucoup plus importantes que le moxisylyte et ont donc un intérêt dans le traitement de l'hypertension artérielle ou des troubles vasculaires périphériques. Les nouveaux produits décrits dans le présent brevet présentent par rapport aux produits de l'art antérieur l'avantage de posséder, de façon tout à fait inattendue, à côté d'une activité alpha-1 bloquante post-synaptique spécifique se manifestant à de faibles doses, une activité inhibitrice de l'angiotensine I présentant un grand intérêt pour leur emploi dans le traitement de l'hypertension artérielle. De plus, ces nouveaux produits, du fait de leur formule symétrique, présentent l'avantage de pouvoir être fabriqués par une méthode beaucoup plus rapide que les produits de l'art antérieur.

Les produits faisant l'objet de la présente invention sont préparés de façon générale par une réaction mettant en jeu un aminoéthoxy-4 isopropyl-5 méthyl-5 phénol de formule générale

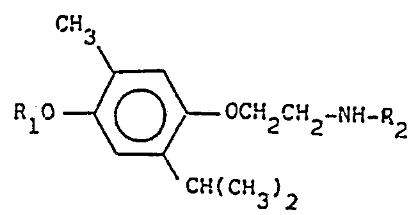
(IV)

dans laquelle $R_1$ représente H, $CH_3$ ou $COCH_3$ et $R_2$ représente H ou $CH_3$ et un halogénoéthoxy-4 isopropyl-5 méthyl-2 phénol de formule générale

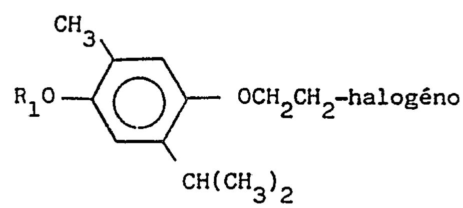
(V)

dans laquelle $R_1$ représente H, $CH_3$ ou $COCH_3$ et halogéno représente un substituant chloro, bromo ou iodo, de préférence bromo, en présence d'un solvant, de préférence l'éthanol, à la température d'ébullition du solvant.

La présente invention va être décrite de façon plus précise dans l'exemple suivant sans toutefois que celui-ci ne limite sa portée.

*Exemple:*

*Chlorhydrate de la bis N,N-(hydroxy-4 isopropyl-2 méthyl-5 phénoxy)-2 N-méthylamine ou COR28 35; chlorhydrate du produit de formule (I) dans laquelle $R_1$ = H et $R_2$ = $CH_3$*

Le mélange constitué par 4 g d'isopropyl-5 méthyl-2 méthylaminoéthoxy-4 phénol, 3,27 g de bromoéthoxy-4 isopropyl-5 méthyl-2 phénol et 50 ml d'éthanol est chauffé au reflux sous agitation. La réaction est suivie en chromatographie sur couches minces. Après neutralisation par un équivalent de soude diluée 1 N, le solvant est évaporé. Le résidu est repris par du chloroforme; la phase

chloroformique est lavée à l'eau, séchée et le solvant est éliminé par évaporation. Le produit obtenu est purifié sur colonne de silice dans le chloroforme pur (éluant chloroforme/méthanol à 5%); les solvants des fractions sont évaporés et les résidus sont repris dans l'éther. Le chlorhydrate est préparé en faisant barboter un courant d'acide chlorhydrique gazeux dans la solution. Rendement 33%.

Caractéristiques physicochimiques: point de fusion 151° C.

Spectre de RMN dans le $DMSOD_6$: 1,1 ppm, 12 protons, doublet, 4 $CH_3$ isopropyliques; 2,1 ppm, 6 protons, singulet, 2 $CH_3$ sur les cycles aromatiques; 2,8-4,6 ppm, 13 protons, massif complexe, $-OCH_2CH_2N(CH_3)CH_2CH_2O-$ + 2 $-CH-$ isopropyliques; 6,5-6,9 ppm, 4 protons, pic large, protons aromatiques; 8,8 ppm, 2 protons, dôme, 2 OH; 11,6 ppm, 1 proton, dôme, $^{+}NH$.

Les produits faisant l'objet de la présente invention ont été soumis à des tests toxicopharmacologiques dont nous exposons les principaux résultats ci-après.

*Toxicité:*

Le COR28 35 administré par voie orale à la dose de 300 mg/kg chez la souris n'induit aucune mortalité. Administré par voie intrapéritonéale chez la souris, il n'induit aucune mortalité à la dose de 500 mg/kg et induit 40% de mortalité à la dose de 1000 mg/kg. Sa DL50 a pu être déterminée pour une administration intraveineuse chez la souris; elle est dans ce cas égale à 53,5 (40,3-71,0) mg/kg.

*Activité alphabloquante:*

In vitro, l'activité alphabloquante est appréciée par la détermination de l'antagonisme des contractions de strips aortiques isolés de lapin induites par la noradrénaline employée à la concentration de $2{,}10^{-6}$ mole/l selon une technique dérivée de celle de Furchgott et Bhadrakom (J. Pharmacol., 1953, 108, 129-43). L'activité maximale est obtenue quand le COR28 35 est introduit dans le bain 60 min avant l'agoniste. La CI50 (concentration inhibant 50% des contractions) est égale dans ces conditions à $1{,}52.10^{-7}$ ($1{,}12.10^{-7}$-$2{,}07.10^{-7}$) mole/l.

In vivo, l'activité alphabloquante est appréciée par la détermination de l'antagonisme de l'hypertension induite par la phényléphrine. Le produit à tester est administré par voie intraveineuse à des rats amyélés et bivagotomisés selon la technique de J.S. Gillespie et T.S. Muir (Br. J. Pharmac. Chemother., 1967, 30, 78-87). On indique ci-après pour le COR28 35, en fonction de la dose administrée, l'activité maximale et le temps au bout duquel l'activité résiduelle est égale à 50% de l'activité maximale: $4{,}42\cdot10^{-7}$ M/kg, −77,8%, 30 mn; $10^{-6}$ M/kg, −94,5%, 55 mn; $3{,}53\cdot10^{-6}$ M/kg, −97,1%, 90 mn. La DE50 déterminée dans ces conditions pour le COR28 35 est de $1{,}48\cdot10^{-8}$ mole/kg au bout de 2 mn, $6{,}77\cdot10^{-7}$ mole/kg au bout de 30 mn et $1{,}87\cdot10^{-6}$ mole/kg au bout de 60 mn.

L'activité alphabloquante des produits selon la présente invention a également été déterminée chez le rat Sprague-Dawley normal vigile. Les veines jugulaires et les artères carotides des animaux sont cathétérisées sous anesthésie à l'éther. Après récupération d'une heure, la pression artérielle et la fréquence cardiaque sont enregistrées de façon continue. L'activité des produits à tester, administrés par voie orale, vis-à-vis de l'effet hypertenseur induit par $4\cdot10^{-3}$ mg/kg de phényléphrine est déterminée. On indique ci-après pour le COR28 35, en fonction de la dose administrée, l'activité maximale et le temps au bout duquel l'activité résiduelle est égale à 50% de l'activité maximale: 5 mg/kg, −38,75%, 66 mn; 10 mg/kg, −37,18%, 65 mn; 20 mg/kg, −66,37%, 66 mn.

*Activité antihypertensive:*

Le produit à tester est administré par voie orale à des rats spontanément hypertendus. La pression artérielle est mesurée de façon indirecte en utilisant un sphygmomanomètre. Pour le COR28 35 administré à la dose de 100 mg/kg/j pendant 2 jours, la chute de pression maximale observée est de 14% le premier jour de traitement, 1 heure après le gavage. La pression est significativement abaissée par rapport au lot témoin 6 heures après le gavage le deuxième jour de traitement. Administré dans les mêmes conditions à la dose de 50 mg/kg/j, la chute de pression artérielle maximale est de 15% 1 heure après le gavage.

L'activité inhibitrice des produits de la présente invention vis-à-vis des contractions induites par $2{,}5\cdot10^{-8}$ g/ml d'angiotensine I sur des préparations de segments d'iléon de cobaye a été déterminée. L'angiotensine I est introduite dans le bain 2 mn après le produit à tester. Dans ces conditions, le COR28 35 entraîne 51% d'inhibition des contractions à la concentration de $10^{-7}$ g/ml.

*Activité antiallergique:*

Dans le test de l'anaphylaxie passive cutanée, le COR28 35 administré à 5 mg/kg par voie orale chez le rat entraîne 60% d'activité. La prométhazine, administrée dans les mêmes conditions, entraîne 53% d'activité à la dose de 10 mg/kg.

*Activité antihistaminique:*

Le COR28 35 inhibe in vitro la contraction induite par $10^{-8}$ g/ml d'histamine sur iléon isolé de cobaye. L'effet maximum est obtenu quand le COR28 35 est mis au contact de l'organe isolé 20 mn avant d'introduire l'agoniste. La CI50 (ou concentration inhibant 50% des contractions) est dans ces conditions de $3{,}34\cdot10^{-7}$ ($2{,}85\cdot10^{-7}$-$3{,}89\cdot10^{-7}$) mole/l.

*Activité antisérotoninergique:*

Le COR28 35 inhibe in vitro la contraction induite par $5\cdot10^{-7}$ g/ml de sérotonine sur iléon isolé de cobaye. L'effet maximum est obtenu quand le COR28 35 est mis au contact de l'organe isolé 15 mn avant d'introduire l'agoniste. Le CI50 (ou concentration inhibant 50% des contractions) est dans ces conditions de $1{,}58\cdot10^{-7}$ ($1{,}00\cdot10^{-7}$-$2{,}51\cdot10^{-7}$) mole/l.

*Activité anticholinergique:*

L'activité inhibitrice des produits de la présente invention vis-à-vis des tremblements musculaires produits chez la souris par l'administration de 0,5 mg/kg d'oxotrémorine par voie sous-cutanée est déterminée. L'effet est apprécié une heute après l'administration par voie intrapéritonéale des produits à tester; dans ces conditions, le COR28 35 est actif à la dose de 5 mg/kg.

*Activité hypolipidémiante:*

Chez la souris rendue hypercholestérolémique par un régime riche en cholestérol et acide cholique pendant 7 jours, l'administration de 200 mg/kg de COR28 35 par voie orale, le 6[e] et le 7[e] jour, entraîne une diminution du cholestérol sérique de 17% et une diminution des HP-bétalipoprotéines de 27% accompagnées d'une diminution du rapport VLDL + LDL/cholestérol.

Compte tenu de leurs propriétés pharmacotoxicologiques et en particulier de leurs propriétés alpha-1 bloquante et antihypertensives, les produits faisant l'objet de la présente invention peuvent être employés par exemple dans le traitement de l'hypertension artérielle (seuls ou en association avec un diurétique ou d'autres médicaments antihypertenseurs), dans le traitement du glaucome ou dans le traitement des troubles vasculaires périphériques tels que l'acrocyanose et le syndrome de Raynaud.

Compte tenu de leurs propriétés antiallergiques, antihistaminiques et antisérotoninergiques, les produits de la présente invention peuvent être utilisés dans le traitement des états allergiques.

Compte tenu de leurs propriétés hypolipémiantes, les produits de la présente invention peuvent être utilisés en association avec un régime alimentaire, dans le traitement des hypercholestérolémies et hypertriglycéridémies.

Les doses et schémas thérapeutiques seront fonction du sujet et de l'affection à traiter. Les produits pourront être administrés par voie orale (par exemple sous forme de gélules, comprimés, gouttes buvables), par voie injectable (soluté injectable pour la voie intramusculaire ou la voie intraveineuse; soluté pour perfusion intraveineuse), par voie rectale (suppositoires), par voie locale (collyres, crèmes, pommades, gels). Suivant les indications, la dose quotidienne variera de 1 à 100 mg en 1 à 3 prises pour la voie orale, de 1 à 100 mg en 1 ou 2 prises pour la voie rectale; la dose administrée par voie intraveineuse pourra varier entre 0,1 et 10 mg. Les collyres contiendront 0,05 à 0,5% de principe actif et les pommades, crèmes ou gels contiendront 0,5 à 5% de principe actif.

## Revendications

1. Nouveaux produits de formule générale

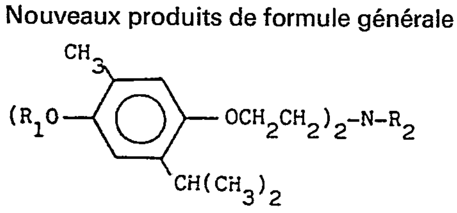

dans laquelle $R_1$ représente H, $CH_3$ ou $COCH_3$ et $R_2$ représente H ou $CH_3$ sous forme de base libre ou de sels pharmaceutiquement compatibles tels que les sels d'addition d'acides minéraux (par exemple acides chlorhydrique, bromhydrique, sulfurique, phosphorique) ou organiques (par exemple acide citrique, méthanesulfonique, camphosulfonique).

2. Nouveaux produits selon la revendication 1, caractérisés en ce que $R_1$ = H et $R_2$ = $CH_3$, sous forme de base libre ou de sels pharmaceutiquement compatibles.

3. Méthode de préparation des produits selon les revendications 1 ou 2, caractérisée en ce que l'on fait réagir un aminoéthoxy-4 isopropyl-5 méthyl-2 phénol de formule générale

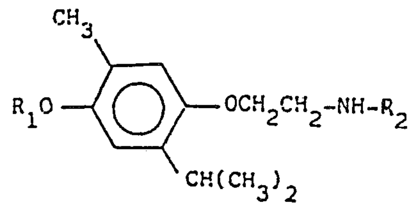

dans laquelle $R_1$ représente H, $CH_3$ ou $COCH_3$ et $R_2$ représente H ou $CH_3$ et un halogénoéthoxy-4 isopropyl-5 méthyl-2 phénol de formule générale

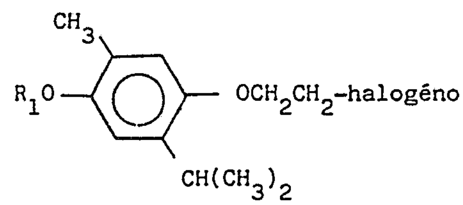

dans laquelle $R_1$ représente H, $CH_3$ ou $COCH_3$ et halogéno représente un substituant chloro, brome ou iodo, en présence d'un solvant, à la température d'ébullition du solvant.

4. Nouveaux médicaments utiles en thérapeutique humaine ou vétérinaire contenant une quantité thérapeutiquement efficace d'au moins un composé selon les revendications 1 ou 2.

5. Nouveaux médicaments utiles en thérapeutique cardiovasculaire contenant une quantité thérapeutiquement efficace d'au moins un composé selon les revendications 1 ou 2.

6. Nouveaux médicaments utiles en thérapeutique antiallergique contenant une quantité thérapeutiquement efficace d'au moins un composé selon les revendications 1 ou 2.

7. Nouveaux médicaments utiles en thérapeutique hypolipémiante contenant une quantité thérapeutiquement efficace d'au moins un composé selon les revendications 1 ou 2.

8. Composition pharmaceutique ou vétérinaire, caractérisée en ce qu'elle contient à titre de principe actif au moins un produit selon les revendications 1 ou 2 en association avec un véhicule pharmaceutique ou un excipient approprié.

## Patentansprüche

1. Neue Verbindungen der allgemeinen Formel

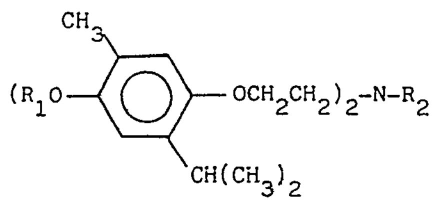

in der $R_1$ H, $CH_3$ oder $COCH_3$ und $R_2$ H oder $CH_3$ bedeuten, in Form ihrer freien Basen oder pharmazeutisch verträglichen Additionssalze mit Mineralsäuren (z.B. Salzsäure, Bromwasserstoffsäure, Schwefelsäure, Phosphorsäure) oder organischen Säuren (z.B. Citronensäure, Methansulfonsäure, Camphosulfonsäure).

2. Neue Verbindungen nach Anspruch 1, dadurch gekennzeichnet, dass $R_1$ = H und $R_2$ = $CH_3$ bedeuten, in Form ihrer freien Basen oder pharmazeutisch verträglichen Salzen.

3. Verfahren zur Herstellung von Verbindungen nach Anspruch 1 oder 2, dadurch gekennzeichnet, dass man ein Aminoethoxy-4-isopropyl-5-methyl-2-phenol der allgemeinen Formel

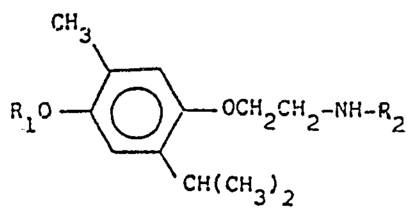

in der $R_1$ H, $CH_3$ oder $COCH_3$ und $R_2$ H oder $CH_3$ bedeuten, und ein Halogenoethoxy-4-isopropyl-5-methyl-2-phenol der allgemeinen Formel

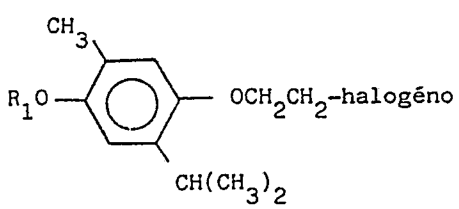

in der $R_1$ H, $CH_3$ oder $COCH_3$ und Halogeno einen Chlor-, Brom- oder Jodsubstituenten bedeuten, in Gegenwart eines Lösemittels bei der Siedetemperatur des Lösemittels reagieren lässt.

4. Neue Arzneimittel zur Verwendung in der Therapie von Menschen oder Tieren, enthaltend eine therapeutisch wirksame Menge mindestens einer der Verbindungen gemäss den Ansprüchen 1 oder 2.

5. Neue Arzneimittel zur Verwendung in der cardiovaskulären Therapie, enthaltend eine therapeutisch wirksame Menge mindestens einer der Verbindungen gemäss Anspruch 1 oder 2.

6. Neue Medikamente zur Verwendung in der antiallergischen Therapie, enthaltend eine therapeutische Menge mindestens einer der Verbindungen gemäss Anspruch 1 oder 2.

7. Neue Medicament zur Verwendung in der hypolipämischen Therapie, enthaltend eine therapeutisch wirksame Menge mindestens einer der Verbindungen gemäss Anspruch 1 oder 2.

8. Pharmazeutische oder veterinärmedizinische Zusammensetzung, dadurch gekennzeichnet, dass sie als Wirkstoff mindestens eine Verbindung gemäss den Ansprüchen 1 oder 2 zusammen mit einem pharmazeutischen Träger oder geeigneten Exzipienten enthält.

## Claims

1. New products of the general formula:

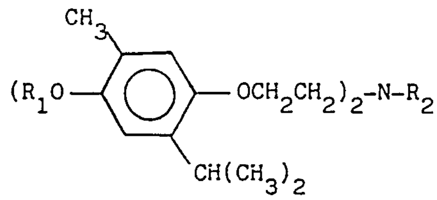

wherein $R_1$ represents H, $CH_3$ or $COCH_3$ and $R_2$ represents H or $CH_3$, as free base or as pharmaceutically acceptable salts, such as addition salts of mineral acids (for example hydrochloric, hydrobromic, sulfuric, phosphoric acids) or of organic acids (for example citric, methanesulfonic, camphorsulfonic acids).

2. New products according to claim 1, characterized in that $R_1$ = H and $R_2$ = $CH_3$, as a free base or pharmaceutically acceptable salts.

3. A process for preparing the products according to claim 1 or 2, characterized in that a 4-aminoethoxy-5-isopropyl-2-methyl phenol of the general formula:

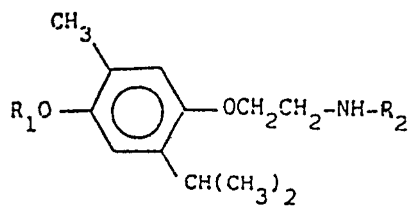

wherein $R_1$ represents H, $CH_3$ or $COCH_3$ and $R_2$ represents H or $CH_3$ is reacted with a 4-halogenoethoxy-5-isopropyl-2-methyl phenol of the general formula:

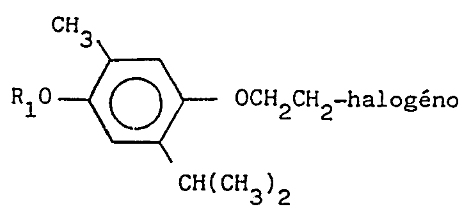

wherein $R_1$ represents H, $CH_3$ or $COCH_3$ and halogeno represents a chloro, bromo or iodo substituent in the presence of a solvent at the boiling point of the solvent.

4. New drugs useful in human or veterinary therapies containing a therapeutically effective amount of at least one compound according the claim 1 or 2.

5. New drugs useful in cardiovascular therapy containing a therapeutically effective amount of at least one compound according to claim 1 or 2.

6. New drugs useful in antiallergic therapy containing a therapeutically effective amount of at least one compound according the claim 1 or 2.

7. New drugs useful in hypolipidemient therapy containing a therapeutically effective amount of at least one compound according the claim 1 or 2.

8. Pharmaceutical or veterinary composition, characterized by containing as active agent at least one product according to claim 1 or 2 in association with a suitable pharmaceutical vehicle or excipient.